# EUROPEAN PATENT SPECIFICATION

(11) **EP 1 027 892 B1**
(45) Date of publication and mention of the grant of the patent: **17.08.2005**
(21) Application number: 99309405.1
(22) Date of filing: 25.11.1999
(51) Int. Cl.: A61K 31/70, A61P 1/08

(54) **Azithromycin combination for emesis control in dogs**
Azithromycin enthaltende Zusammensetzungen zur Kontrolle von Emesis bei Hunden
Composition contenant de l' azithromycine pour le contrôle de l'émèse chez les chiens

(30) Priority: 29.01.1999 US 117876
(43) Date of publication of application: 16.08.2000
(73) Proprietor: Pfizer Products Inc., Groton, Connecticut 06340 (US)
(72) Inventor: Darrington, Richard Todd, Uncasville, Connecticut 06382 (US); Godek, Dennis Michael, Glastonbury, Connecticut 06033 (US); Illyes, Elizabeth Leigh Fraering, Marshall, Illinois 62441 (US)
(74) Representative: Stuart, Peter Graham

(56) References cited:
- EP-A- 0 533 280
- EP-A- 0 627 221
- EP-A- 0 773 026
- US-A- 5 900 257
- LONGMORE J ET AL: "Neurokinin-receptor antagonists: Pharmacological tools and therapeutic drugs." CANADIAN JOURNAL OF PHYSIOLOGY AND PHARMACOLOGY, vol. 75, no. 6, 1997, pages 612-621, XP001068704 ISSN: 0008-4212
- QUARTARA L ET AL: "The tachykinin NK1 receptor. Part II: Distribution and pathophysiological roles." NEUROPEPTIDES, vol. 32, no. 1, February 1998 (1998-02), pages 1-49, XP001068351 ISSN: 0143-4179
- ROSEN T J ET AL: "Synthesis and structure-activity relationships of CP-122,721, a second-generation NK-1 receptor antagonist" BIOORGANIC & MEDICINAL CHEMISTRY LETTERS, OXFORD, GB, vol. 8, no. 3, 3 February 1998 (1998-02-03), pages 281-284, XP004136864 ISSN: 0960-894X
- GARDNER C J ET AL: "THE BROAD-SPECTRUM ANTI-EMETIC ACTIVITY OF THE NOVEL NON-PEPTIDE TACHYKININ NK1 RECEPTOR ANTAGONIST GR203040" BRITISH JOURNAL OF PHARMACOLOGY, BASINGSTOKE, HANTS, GB, vol. 116, no. 8, 1995, pages 3158-3163, XP000575088 ISSN: 0007-1188
- DATABASE BIOSIS [Online] BIOSCIENCES INFORMATION SERVICE, PHILADELPHIA, PA, US; 1993 QIN XIN YU ET AL: "Effects of cholinoceptor and 5-hydroxytryptamine-3 receptor antagonism on erythromycin-induced canine intestinal motility disruption and emesis." Database accession no. PREV199395089139 XP002196779 & BRITISH JOURNAL OF PHARMACOLOGY, vol. 108, no. 1, 1993, pages 44-49, ISSN: 0007-1188

## Description

### Field of Invention

This invention relates to methods and compositions useful in the treatment of cancer or bacterial or protozoal infections in mammals. The invention further relates to a method of preventing or reducing emesis in mammals associated with macrolide antibiotics.

### Background of the Invention

Macrolide antibiotics such as erythromycin and azithromycin are useful in the treatment of a broad spectrum of bacterial infections and protozoa infections in mammals, fish and birds, and may also be useful in the treatment of cancer. Unfortunately, macrolide antibiotics tend to cause emesis in companion animals. *See, e.g.*, Hall, J.A., and Washabau, R.J., *Small Animal Gastroent.* 19(3):261-268 (1997); Kunkle, G.A., *et al., J. Am. Animal Hosp. Assn.* 31:46-54 (1995). When a macrolide antibiotic is administered subcutaneously or intravenously, this attendant emesis causes unwanted pain and suffering in the companion animal. When a macrolide antibiotic is administered orally, however, its emetic effect can interfere with the treatment of the companion animal. This is because it can be difficult to determine how much of the antibiotic was effectively administered to the animal prior to vomiting.

As the abilities of bacteria and protozoa to resist antibiotics increase, stronger, more effective antibiotics are required to prevent and treat bacteria and protozoa infections in companion animals. There thus exists a need for a method of safely and effectively administering macrolide antibiotics to these animals.

Antiemetic drugs are typically effective against only one or a few emetic stimuli. For example, 5-hydroxytryptamine antagonists are only effective against vomiting elicited by ipecac, radiation, acute cancer chemotherapy and, to a lesser extent, recovery from anesthesia. Lucot, J.B., *et al., British J. Pharmacol.* 120(1):l16-120 (1997). Similarly, phenothiazines are generally not useful in treating motion sickness. *The Merck Manual* p. 1281 (16^{th} ed., 1992). It is also well known that antiemetic drugs are not equally effective in preventing or reducing emesis in different species of animals. Lucot, J.B., *et al., British J. Pharmacol.* 120(1):116-120 (1997).

It has recently been alleged that some Substance P receptor antagonists are effective in reducing cisplatin- and radiation-induced emesis in ferrets. United States Patent No. 5,547,964. Substance P is a pharmacologically active neuropeptide belonging to the tachykinin family of peptides. It possesses a characteristic amino acid sequence disclosed by United States Patent No. 4,680,283. Mammalian tachykinins are widely distributed throughout the central and peripheral nervous systems, where they act as neurotransmitters or neuromodulators. They have been implicated in a wide variety of biological actions such as pain transmission, neurogenic inflammation, smooth muscle contraction, vasodilation, secretion, and activation of the immune system. The effects of tachykinins are mediated by the G-protein-coupled receptors NK₁, NK₂ and NK₃, and may thus be controlled by antagonists of these receptors. The development of non-peptide receptor antagonists has revealed, however, pharmacological differences across species. Longmore, J., *et al., Can. J. Physiol. Pharmacol*. 75(6):612-21 (1997).

A particular tachykinin receptor antagonist is CP122,721, chemically named (2S,3S)-3-(2-methoxy-5-trifluoromethoxybenzyl)amino-2-phenylpiperidine. CP122,721 is a tachykinin NK, receptor antagonist that reportedly reduces vomiting in ferrets caused by intragastric administration of copper sulfate and ipecac syrup, subcutaneous administration of loperamide, and intraperitoneal administration of cisplatin. Its effectiveness reportedly varies with emetic stimuli. Gonsalves, S., *et al., European J. Pharmacol.* 305:181-185 (1996).

### Summary of the Invention

The present invention is directed to the use of a pharmaceutically effective amount of a macrolide antibiotic and a pharmaceutically effective amount of a substance P antagonist for the preparation of medicament for the treatment of cancer or a bacterial or protozoal infection in a subject in need of such treatment. Preferably, the subject is a mammal; more preferably, the subject is a companion animal.

Macrolide antibiotics suitable for use in this method include erythromycin, clarithromycin, azithromycin, josamycin, and tylosin.

Substance P antagonists suitable for use in this method include:
(2S,3S)-3-(5-tert-butyl-2-methoxybenzyl)amino-2-(3-trifluromethoxylphenyl)piperidine;
(2S,3S)-3-(2-isopropoxy-5-trifluoromethoxybenzyl)amino-2-phenylpiperidine;
(2S,3S)-3-(2-ethoxy-5-trifluoromethoxybenzyl)amino-2-phenylpiperidine;
(2S,3S)-3-(2-methoxy-5-trifluoromethoxybenzyl)amino-2-phenylpiperidine;
(2S,3S)-3-(5-tert-butyl-2-trifluoromethoxybenzyl)amino-2-phenylpiperidine;
2-(diphenylmethyl)-N-(2-methoxy-5-trifluoromethoxyphenyl)methyl-1-azabicyclo[2.2.2]octan-3-amine;
(2S,3S)-3-[5-chloro-2-(2,2,2-trifluoroethoxy)-benzyl]amino-2-phenylpiperidine;
(2S,3S)-3-(2-difluoromethoxy-5-trifluoromethoxybenzyl)amino-2-phenylpiperidine;
(2S,3S)-2-phenyl-3-[2-(2,2,2-trifluoroethoxybenzyl)]aminopiperidine;
(2S,3S)-2-phenyl-3-(2-trifluoromethoxybenzyl)aminopiperidine;
3-[N-(2-methoxy-5-trifluoromethoxybenzyl)-amino]-5,5-dimethyl-2-phenylpyrrolidine;
3-[N-(2-methoxy-5-trifluoromethoxybenzyl)-amino]-4,5-dimethyl-2-phenylpyrrolidine;
3-(2-cyclopropyloxy-5-trifluoromethoxybenzyl)amino-2-phenylpiperidine;
3-(2-cyclopropylmethoxy-5-trifluoromethoxybenzyl)amino-2-phenylpiperidine;
3-(2-difluoromethoxy-5-phenylbenzyl)amino-2-phenylpiperidine;
3-(5-cyclopropylmethoxy-2-difluoromethoxybenzyl)amino-2-phenylpiperidine;
3-(2-methoxybenzyl)amino-2-(3-trifluoromethoxyphenyl)piperidine;
3-(2-methoxy-5-trifluoromethoxybenzyl)amino-2-(3-trifluoromethoxyphenyl)piperidine;
2-phenyl-3-(5-n-propyl-2-trifluoromethoxybenzyl)amino-piperidine;
3-(5-isopropyl-2-trifluoromethoxybenzyl)amino-2-phenylpiperidine;
3-(5-ethyl-2-trifluoromethoxybenzyl)amino-2-phenylpiperidine;
3-(5-sec-butyl-2-trifluoromethoxybenzyl)amino-2-phenylpiperidine;
3-(5-difluoromethoxy-2-methoxybenzyl)amino-2-phenylpiperidine;
3-(2-methoxy-5-trifluoromethoxybenzyl)amino-2-phenylpyrrolidine;
3-(2-methoxy-5-trifluoromethoxybenzyl)amino-2-phenylhomopiperidine;
2-benzhydryl-3-(2-methoxy-5-trifluoromethoxy-benzyl)aminopyrrolidine;
2-benzhydryl-3-(2-methoxy-5-trifluoromethoxy-benzyl)aminohomopiperidine;
3-[2,5-bis-(2,2,2-trifluoroethoxy)benzyl]amino-2-phenylpiperidine;
2-phenyl-3-(3-trifluoromethoxybenzyl)aminopiperidine;
2-benzhydryl-3-(2-methoxy-5-trifluoromethoxybenzyl)aminopiperidine;
1-(5,6-difluorohexyl)-3-(2-methoxy-5-trifluoromethoxybenzyl)amino-2-phenylpiperidine;
1-(6-hydroxyhexyl)-3-(2-methoxy-5-trifluoromethoxybenzyl)amino-2-phenylpiperidine;
3-phenyl-4-(2-methoxy-5-trifluoromethoxybenzyl)amino-2-azabicyclo[3.3.0]octane;
4-benzhydryl-5-(2-methoxy-5-trifluoromethoxybenzyl)amino-3-azabicyclo[4.1.0]heptane;
4-(2-methoxy-5-trifluoromethoxybenzyl)amino-3-phenyl-2-azabicyclo[4.4.0]decane;
2-phenyl-3-(2-methoxy-5-trifluoromethoxybenzyl)aminoquinuclidine;
8-benzhydryl-N-(2-methoxy-5-trifluoromethoxybenzyl)-9-azatricyclo[4.3.1.0^{4,9}]decan-7-amine;
9-benzhydryl-N-(2-methoxy-5-trifluoromethoxybenzyl)-10-azatricyclo[4.4.1.0^{5,10}]undecan-8-amine;
9-benzhydryl-N-(2-methoxy-5-trifluoromethoxybenzyl)-3-thia-10-azatricyclo[4.4.1.0^{5,10}]undecan-8-amine;
8-benzhydryl-N-(2-methoxy-5-trifluoromethoxybenzyl)-9-azatricyclo[4.3.1.0^{4,9}]decan-7-amine;
5,6-pentamethylene-2-benzhydryl-3-(2-methoxy-5-trifluoromethoxybenzyl)aminoquinuclidine;
5,6-trimethylene-2-benzhydryl-3-(2-methoxy-5-trifluoromethoxybenzyl)aminoquinuclidine;
9-benzhydryl-N-((2-methoxy-5-trifluoromethoxyphenyl)-methyl)-3-oxa-10-azatricyclo[4.4.1.0^{5,10}]undecan-3-amine;
8-benzhydryl-N-((2-methoxy-5-trifluoromethoxyphenyl)-methyl)-7-azatricyclo[4.4.1.0^{5,10}]undecan-9-amine; and
2-benzhydryl-N-((2-methoxy-5-trifluoromethoxyphenyl)-methyl)-1-azabicyclo[3.2.2]nonan-3-amine;
and pharmaceutically acceptable salts and solvates thereof.

Preferably, the Substance P antagonist is selected to be (2S,3S)-3-(2-methoxy-5-trifluoromethoxybenzyl)amino-2-phenylpiperidine, or a pharmaceutically acceptable salt or solvate thereof.

According to this use, the amount of macrolide antibiotic administered to the subject is preferably between about 0.2 mg/kg/day and about 200 mg/kg/day, more preferably between about 2 mg/kg/day and about 100 mg/kg/day, and most preferably between about 4 mg/kg/day to about 50 mg/kg/day.

The amount of Substance P antagonist administered to the subject is preferably between about 0.2 mg/kg/day and about 20 mg/kg/day, more preferably between about 1 mg/kg/day and about 10 mg/kg/day, and most preferably between about 2 mg/kg/day and about 7 mg/kg/day.

The present invention is also directed to the use of a pharmaceutically effective amount of a substance P antagonist for the preparation of medicament for the prevention or treatment of emesis associated with a macrolide antibiotic in a subject in need of such prevention or treatment. The medicament may be used before, concurrently with, or after administration of a macrolide antibiotic. Preferably, the subject is a mammal; more preferably, the subject is a companion animal. It is preferred that the Substance P antagonist be selected from the group described above.

According to this use, the Substance P antagonist is preferably administered before or concurrently with a macrolide antibiotic. More preferably, the Substance P antagonist is administered concurrently with the macrolide antibiotic.

If the Substance P antagonist is administered before a macrolide antibiotic, it is preferably administered less than about 2 hours before, more preferably less than about 1 hour before, and most preferably less than about 0.5 hours before a macrolide antibiotic.

If the Substance P antagonist is administered after a macrolide antibiotic, it is preferably administered less than about 1 hour after, more preferably less than about 0.5 hour after, and most preferably less than about 0.25 hours after a macrolide antibiotic.

In one embodiment of this use, the amount of Substance P antagonist administered to the subject is selected to be between about 0.2 mg/kg/day and about 20 mg/kg/day, more preferably between about 1 mg/kg/day and about 10 mg/kg/day, and most preferably between about 2 mg/kg/day and about 7 mg/kg/day.

This invention further encompasses a composition comprising a macrolide antibiotic and a Substance P antagonist. It is preferred that the macrolide antibiotic and Substance P antagonist used in the composition be selected from the groups described above.

Another embodiment of the invention is directed to a pharmaceutical composition comprising a pharmaceutically effective amount of a macrolide antibiotic, a pharmaceutically effective amount of a Substance P antagonist, and a carrier. This pharmaceutical composition is useful in the treatment of cancer or bacterial or protozoal infections in a subject in need of such treatment. Preferably, the subject is a mammal; more preferably, the subject is a companion animal.

The pharmaceutical compositions of this invention are suitable for oral, rectal, parenteral (intravenous, intramuscular), transdermal, buccal, nasal, sublingual, or subcutaneous administration. Dosage forms of the compositions of this invention include tablets, trochees, dispersions, suspensions, solutions, capsules, and patches.

### Definitions

As used herein, the term "mammal" includes, but not limited to, a companion animal and a human.

As used herein, the term "companion animal" includes, but is not limited to, a dog and a cat.

As used herein the terms "bacterial infection(s)" and "protozoal infection(s)" include bacterial infections and protozoal infections that occur in mammals, fish and birds as well as disorders related to bacterial infections and protozoal infections that may be treated or prevented by the administration of antibiotics. Such bacterial infections and protozoal infections, and disorders related to such infections, include the following: pneumonia, otitis media, sinusitis, bronchitis, tonsillitis and mastoiditis related to infection by *Staphylococcus pneumoniae, Haemophilus influenzae, Moraxella catarrhalis, Staphlococcus aureus,* or *Peptostreptococcus spp.*; pharynigis, rheumatic fever and glomerulonephritis related to infection by *Streptococcus pyogenes,* Groups C and G streptococci, *Clostridium diptheriae,* or *Actinobacillus haemolyticum;* respiratory tract infections related to infection by *Mycoplasma pneumoniae, Legionella pneumophila, Streptococcus pneumoniae, Haemophilus influenzae,* or *Chlamydia pneumoniae;* uncomplicated skin and soft tissue infections, abscesses and osteomyelitis, and puerperal fever related to infection by *Staphylococcus aureus,* coagulase-positive staphlococci (*i.e., S. Epidermis., S. Hemolyticus,* etc.), *Staphylococcus pyogenes, Streptococcus agalactiae,* Streptococcal groups C-F (minute-colony streptococci), viridans streptococci, *Corynebacterium minutissimum, Clostridium* spp., or *Bartonella henselae;* uncomplicated acute urinary tract infections related to infection by *Staphylococcus saprophyticus* or *Enterococcus* spp.; urethritis and cervicitis; and sexually transmitted diseases related to infection by *Chlamydia trachomatis, Haemophilus ducreyi, Treponema* *pallidum, Ureaplasma urealyticum,* or *Neiserria gonorrhea;* toxin diseases related to infection by *S. Aureus* (food poisoning and Toxic Shock Syndrome), or Groups A, B and C streptococci; ulcers related to infection by *Helicobacter pylori;* systemic febrile syndromes related to infection by *Borrelia recurrentis;* Lyme disease related to infection by *Borrelia burgdorferi;* conjunctivitis, keratitis, and dacrocystitis related to infection by *Chlamydia trachomatis, Neisseria gonorrhoeae, S. Aureus, S. Pneumoniae, S. Pyogenes, H. Influenzae,* or *Listeria* spp.; disseminated *Mycobacterium avium complex* (MAC) disease related to infection by *Mycobacterium avium,* or *Mycobacterium intracellulare;* gastroenteritis related to infection by *Campylobacter jejuni*; intestinal protozoa related to infection by *Cryptosporidium* spp.; odontogenic infection related to infection by viridans streptococci; persistent cough related to infection by *Bordetella pertussis;* gas gangrene related to infection by *Clostridium perfringens* or *Bacteroides* spp.; and atherosclerosis related to infection by *Helicobacter pylori* or *Chlamydia pneumoniae;* bovine respiratory disease related to infection by *P.haem., P. Multocida, Mycoplasma bovis,* or *Bordetella* spp.; cow enteric disease related to infection by *E. Coli* or protozoa (*i.e.,* coccidia, cryptosporidia, etc.); dairy cow mastitis related to infection by *Staph. Aureus, Strep. Uberis, Strep. Agalactiae, Strep. Dysgalactiae, Klebsiella* spp., *Corynebacterium,* or *Enterococcus* spp.; swine respiratory disease related to infection by *A. Pleuro., P. Multocida* or *Mycoplasma* spp.; swine enteric disease related to infection by *E. Coli, Lawsonia intracellularis, Salmonella,* or *Serpulina hyodyisinteriae;* cow footrot related to infection by *Fusobacterium* spp.; cow metritis related to infection by *E. Coli;* cow hairy warts related to infection by *Fusobacterium necrophorum* or *Bacteroides nodosus;* cow pink-eye related to infection by *Moraxella bovis;* cow premature abortion related to infection by protozoa (*i.e.*, neosporium) urinary tract infection in dogs and cats related to infection by *E. Coli;* skin and soft tissue infections in dogs and cats related to infection by *Staph. Epidermidis, Staph. Intermedius, coagulase neg. Staph.* Or *P. Multocida;* and dental or mouth infections in dogs and cats related to infection by *Alcaligenes* spp., *Bacteroides* spp., *Clostridium* spp., *Enterobacter* spp., *Eubacterium, Peptostreptococcus, Porphyromonas,* or *Prevotella.* Other bacterial infections and protozoal infections and disorders related to such infections that may be treated or prevented in accord with the methods of the invention are referred to in Sanford, J.P., *et al.,* "The Sanford Guide To Antimicrobial Therapy," 27^{th} Edition (Antimicrobial Therapy, Inc., 1996).

As used herein, the term "treating cancer or a bacterial or protozoal infection" includes preventing, reducing, and alleviating the symptoms of cancer and the symptoms of a bacterial or protozoal infection.

As used herein, the term "treating emesis" includes reducing and eliminating emesis.

As used herein, the term "adjunctive administration" when used to describe the administration of two or more compounds to a subject means that the compounds, which may be administered by same or different routes, are administered concurrently (*e.g.*, as a mixture) or sequentially such that pharmacological effects of each overlap in time. When the term "adjunctive administration" is used to describe the administration of a macrolide antibiotic and a Substance P antagonist, the term preferably means that if the Substance P antagonist is administered before the macrolide antibiotic, it is administered less than about 2 hours before, more preferably less than about 1 hour before, and most preferably less than about 0.5 hours before the macrolide antibiotic; and if the Substance P antagonist is administered after the macrolide antibiotic, it is administered less than about 1 hour after, more preferably less than about 0.5 hour after, and most preferably less than about 0.25 hours after the macrolide antibiotic.

As used herein, the term "associated with a macrolide antibiotic" when used to describe emesis includes emesis that is caused, worsened or prolonged by a macrolide antibiotic or a metabolite thereof.

As used herein, the term "macrolide antibiotic" means a compound having a macrolide structure that is effective in the prevention or treatment of cancer or a bacteria or protozoal infection in a subject. Preferably, the subject is a mammal; more preferably, the subject is a companion animal. The term includes, but is not limited to: erythromycin; clarithromycin; azithromycin; josamycin; tylosin; compounds disclosed by United States Patent Nos. 2,653,899; 3,417,077; 4,328,334; 4,474,768; and 4,517,359; and those disclosed by United States Provisional Application Nos. 60/046150 filed May 9, 1997; 60/049348 filed June 11, 1997; 60/049349 filed June 11, 1997; 60/054866 filed August 6, 1997; and 60/063676 filed October 29, 1997.

As used herein, the term "pharmaceutically acceptable amount of a macrolide antibiotic" means an amount of a macrolide antibiotic that when administered to a subject is effective in preventing or reducing cancer or a bacteria or protozoal infection. Preferably, the subject is a mammal; more preferably, the subject is a companion animal.

As used herein, the term "Substance P antagonist means a compound or mixture of compounds that reduces or minimizes the physiological effects of Substance P, and emesis in particular. The term includes compounds disclosed by United States Patent Nos. 5,547,964 and 5,773,450; by published European Patent Application Nos. 512901, 512902, 514273, 514275, 517589, 520555, 522808, 528495, 532456; and by published PCT Patent Application Nos. 92/15585, 92/17449, 92/20661, 92/20676, 92/21677, 92/22569, 93/00331, 93/01159, 93/01160, 93/01165, 93/01169, and 93/01170.

As used herein, the term "pharmaceutically effective amount of a Substance P antagonist" means an amount of a Substance P antagonist that when administered to a subject is effective in preventing or reducing emesis in a subject that is caused, worsened, or prolonged by a macrolide antibiotic. Preferably, the subject is a mammal; more preferably, the subject is a companion animal.

As used herein, the term "pharmaceutically acceptable salt" means a non-toxic acid addition salt, i.e., a salt containing a pharmacologically acceptable anion such as, but not limited to, hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate (*i.e.,* 1,1'-methylene-bis-(2-hydroxy-3-naphthoate)).

### Detailed Description of the Invention

This invention is based upon the discovery that Substance P antagonists are unusually effective in preventing or treating emesis in mammals that is caused, worsened or prolonged by macrolide antibiotics such as erythromycin, clarithromycin, azithromycin, josamycin, and tylosin. Such emesis is particularly common in companion animals, and the Substance P antagonist CP122,721 has been found to be particularly effective in its prevention and reduction in these animals.

This invention thus encompasses a the use of substance P antagonists for preventing or treating emesis associated with a macrolide antibiotic. According to this use a pharmaceutically effective amount of at least one Substance P antagonist is administered to a subject in need of such prevention or treatment before, concurrently with, or after administration of a macrolide antibiotic. It is preferred that the Substance P antagonist be administered before or concurrently with the macrolide antibiotic.

The invention is also directed to a use for treating cancer or a bacterial or protozoa infection. The method comprises the adjunctive administration of a macrolide antibiotic and a Substance P antagonist to a subject in need of such treatment. According to this method, the macrolide antibiotic and Substance P antagonist may be administered by the same or different routes. For example, both may be administered orally; one may be administered orally while the other is administered intravenously; or one may be administered orally while the other is administered subcutaneously. All possible combinations of administrative routes known to those skilled in the art are incorporated in the invention, although oral routes are preferred. If a Substance P antagonist is administered concurrently with a macrolide antibiotic, however, it is preferred that they be administered by the same route.

This invention further encompasses a composition comprising a macrolide antibiotic and a Substance P antagonist. The invention also encompasses a pharmaceutical composition comprising a pharmaceutically effective amount of a macrolide antibiotic, a pharmaceutically effective amount of a Substance P antagonist, and a carrier. The pharmaceutical composition is useful in the prevention and treatment of cancer or bacteria or protozoal infections in mammals.

The preparation of macrolide antibiotics suitable for use in the medical uses and compositions of the invention is disclosed by, for example, United States Patent Nos. 2,653,899; 3,417,077; 4,328,334; 4,474,768; and 4,517,359, and by United States Provisional Application Nos. 60/046150 filed May 9, 1997; 60/049348 filed June 11, 1997; 60/049349 filed June 11, 1997; 60/054866 filed August 6, 1997; and 60/063676 filed October 29, 1997.

The preparation of Substance P antagonists suitable for use in the medical uses and compositions of the invention is disclosed by, for example, United States Patent Nos. 5,547,964 and 5,773,450 by published European Patent Application Nos. 512901, 512902, 514273, 514275, 517589, 520555, 522808, 528495, 532456; and by published PCT Patent Application Nos. 92/15585, 92/17449, 92/20661, 92/20676, 92/21677, 92/22569, 93/00331, 93/01159, 93/01160, 93/01165, 93/01169, and 93/01170. It is preferred that the Substance P antagonist used in the compositions of this invention is CP122,721.

### Pharmaceutical Formulations and Methods of Treatment

The compounds of this invention (macrolide antibiotics and Substance P antagonists; hereinafter also referred to as "the active compounds"), may be administered through oral, parenteral, topical, or rectal routes. In general, the macrolide antibiotics are most desirably administered in dosages ranging from about 0.2 mg per kg body weight per day (mg/kg/day) to about 200 mg/kg/day in single or divided doses (*i.e.*, from 1 to 4 doses per day), although variations will necessarily occur depending upon the species, weight and condition of the subject being treated and the particular route of administration chosen. A dosage level that is in the range of about 2 mg/kg/day to about 100 mg/kg/day is preferred, and a dosage level of macrolide antibiotic that is in the range of about 4 mg/kg/day to about 50 mg/kg/day is most preferred. Variations may nevertheless occur depending upon the species of companion animal being treated and its individual response to the macrolide antibiotic, as well as on the type of pharmaceutical formulation chosen and the time period and interval at which such administration is carried out. In some instances, dosage levels below the lower limit of the aforesaid ranges may be more than adequate, while in other cases still larger doses may be employed without causing any harmful side effects provided that such larger doses are first divided into several small doses for administration throughout the day.

In general, the Substance P antagonists are most desirably administered in dosages ranging from about 0.2 mg per kg body weight per day (mg/kg/day) to about 20 mg/kg/day in single or divided doses (*i.e.,* from 1 to 4 doses per day). A dosage level that is in the range of about 1 mg/kg/day to about 10 mg/kg/day is preferred, and a dosage level of Substance P antagonist that is in the range of about 2 mg/kg/day to about 7 mg/kg/day is most preferred. Variations will necessarily occur, however, depending upon the species, weight and condition of the subject being treated and the particular route of administration chosen. Variations will also occur depending upon the severity of the emetic response caused, worsened or prolonged by the particular macrolide antibiotic administered. Dosage variations may also occur depending upon the type of pharmaceutical formulation chosen and the time period and interval at which administration of the active ingredients is carried out. In some instances, dosage levels below the lower limit of the aforesaid ranges may be more than adequate, while in other cases still larger doses may be employed without causing any harmful side effects, provided that such larger doses are first divided into several small doses for administration throughout the day.

The active compounds may be administered separately or together, and may be administered alone or in combination with pharmaceutically acceptable carriers or diluents by the routes previously indicated. Such administration may be carried out in single or multiple doses. The active compounds may be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents. Moreover, oral pharmaceutical compositions can be suitably sweetened and/or flavored. In general, the active compounds are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Lubricating agents such as magnesium stearate, sodium lauryl sulfate and talc are also useful for tableting purposes. Solid compositions of a similar type may also be employed as fillers in gelatin capsules. Preferred fillers include lactose or milk sugar as well as high molecular weight polyethylene glycols. When aqueous suspensions and/or elixirs are desired for oral administration, the active compound may be combined with various sweetening or flavoring agents, coloring matter or dyes, and, if so desired, emulsifying and/or suspending agents as well, together with diluents such as water, ethanol, propylene glycol, glycerin and various like combinations thereof.

In addition to the common dosage forms set out above, the compounds of the invention may also be administered by controlled release means and/or delivery devices capable of releasing the active compound at the required rate to maintain constant pharmacological activity for a desirable period of time. Such dosage forms provide a supply of a drug to the body during a predetermined period of time and thus maintain drug levels in the therapeutic range for longer periods of time than conventional non-controlled formulations. Suitable controlled release pharmaceutical compositions and delivery devices that may be adapted for the administration of the active compounds of the invention are described by U.S. Patent Nos.: 3,847,770; 3,916,899; 3,536,809; 3,598,123; 3,630,200; 4,008,719; 4,687,610; 4,769,027; 5,674,533; 5,059,595; 5,591,767; 5,120,548 ; 5,073,543; 5,639,476; 5,354,566; and 5,733,566. For example, the active compounds may be coupled to a class of biodegradable polymers useful in achieving controlled release of a drug, for example, polylactic acid, polyglycolic acid, copolymers of polylactic and polyglycolic acid, polyepsion caprolatone, polyhydroxy butyric acid, copolymers of polylactic and polyglycolic acid, polyepsilon caprolactone, polyhydroxy butyric acid, polyorthoesters, polyacetals, polydihydropyrans, polycyanoacrylates and crosslinked or amphipathic block copolymers of hydrogels.

For parenteral administration, solutions of an active compound in either sesame or peanut oil or in aqueous propylene glycol may be employed. The aqueous solutions should be suitably buffered (preferably at a pH of less than about 7) if necessary, and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

It is also possible to administer the active compounds of the invention topically. This may be done by way of creams, jellies, gels, pastes, patches, ointments and the like, in accordance with standard pharmaceutical practice. The active compounds may further be administered in the feed of animals or orally as a drench composition.

The active compounds may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The active compounds may also be coupled with soluble polymers as targetable drug carriers. Such polymers can include polyvinlpyrrolidone, pyran copolymer, polyhydroxypropylmethacrylamide phenyl, polyhydroxyethylaspartamide-phenol, or polyethyleneoxide-polylysine substituted with palmitoyl residues.

### Examples

The following examples further illustrate the methods and compositions of the invention. It is to be understood that this invention is not limited to the specific details of these examples.

### Example 1: Use of CP122,721 to Reduce Azithromycin-Induced Emesis

The ability of various compounds to control azithromycin-induced emesis in dogs was measured in 13 studies using two groups of dogs. The majority of the studies were conducted using a family (dam and offspring) of 12 azithromycin-sensitive English pointers, bred and maintained by Young Veterinary Research Services of Turlock, California. The remaining studies were conducted using random source dogs maintained at the Pfizer Animal Health Research facility in Terre Haute, Indiana. In all studies, azithromycin was administered in immediate release formulations at a dose of 10 mg/kg.

Initially, four commercialized antiemetic products and CP122,721 were screened individually in the azithromycin-sensitive population with a minimum 7 day washout period between antiemetic tests. The results of these tests are provided below in Table 1. Antiemetics were administered orally about 15 to 30 minutes prior to oral treatment with 10 mg/kg azithromycin in an immediate release tablet. Of the antiemetics tested, only CP122,721 was 100% effective in preventing emesis when administered in amount of 5 mg/kg. Ondasetron, a 5-HT3 receptor blocker, was the most effective of the commercial products tested, reducing the incidence of emesis by 58%. Other antiemetics reduced the incidence of emesis by only about 10 to 42%.

**Table 1.**

| Control of Azithromycin-lnduced Emesis in an Azithromycin-sensitive Dog Population | | | | |
|---|---|---|---|---|
| Antiemetic | Class | Drug(mg/kg) | Dose Timing Before Azithromycin (minutes) | Emetic Dogs/Total Dogs |
| None | - | - | - | 12/12 |
| CP122,721 | Substance P antagonist | 5 | 30 | 0/12 |
| Metoclopramide | Benzamide | 0.5 | 15 | 11/12 |
| Dimenhydrinate Dramamine® | Antihistamine | 8 | 30 | 10/12 |
| Prochlorperazine Compazine® | Phenothiazine | 1 | 30 | 7/12 |
| Ondansetron Zofran® | Seratonin 5-HT, receptor blocker | 1 | 30 | 5/12 |

### Example 2: Determination of Suitable Dosages of CP122,721 when Administered Concurrently with Azithromycin

Four studies were conducted to titrate the dose of CP122,721 for maximum antiemetic activity with and without a delay between oral antiemetic and oral azithromycin dosing. In the azithromycin-sensitive pointers, 2.5 mg/kg CP122,721 was effective in preventing emesis when administered 30 minutes prior to azithromycin. When azithromycin was administered simultaneously with 2.5 mg/kg CP122,721, however, all pointers were emetic. While these studies were in progress, random source dogs (8 per treatment) were administered 0, 1.5 or 3 mg/kg CP122,721 simultaneously with 10 mg/kg azithromycin. The incidence of emesis was not reduced in dogs given 1.5 mg/kg CP122,721, but 3 mg/kg CP122,721 was completely effective in preventing azithromycin emesis. In the fourth study, the sensitive pointers received either 3 or 4 mg/kg CP122,721 simultaneously with azithromycin. The incidence of emesis was equivalent with one dog in each treatment group vomiting. The CP122,721 dose and timing titrations are summarized in Table 2.

**Table 2.**

| Dose Titrations of CP122,721 for the Control of Azithromycin-Induced Emesis in Dogs | | | |
|---|---|---|---|
| Dog Population | CP122,721 DOSE (mg/kg) | Dose Timing Before Azithromycin (minutes) | Emetic Dogs/ Total Dogs |
| | | 30 | 4/4 |
| | 1 | 30 | 0/4 |
| Sensitive | | 30 | 0/4 |
| | 2.5 | 30 | 0/6 |
| Sensitive | 2.5 | 0 | 6/6 |
| | 0 | 0 | 5/8 |
| Random | 1.5 | 0 | 4/8 |
| source | 3 | 0 | 0/8 |
| | 3 | 0 | 1/6 |
| Sensitive | 4 | 0 | 1/6 |

The invention is further defined with reference to the claims appended hereto.

## Claims

1. The use of a pharmaceutically effective amount of a macrolide antibiotic and a pharmaceutically effective amount of a substance P antagonist for the preparation of medicament for the treatment of cancer or a bacterial or protozoal infection in a mammalian subject in need of such treatment.

2. The use as claimed in Claim 1 wherein the subject is a companion animal or human.

3. The use as claimed in Claim 1 wherein the macrolide antibiotic is selected to be erythromycin, clarithromycin, azithromycin, josamycin, or tylosin.

4. The use as claimed in Claim 1 wherein the Substance P antagonist is selected from the group consisting of:
(2S,3S)-3-(5-tert-butyl-2-methoxybenzyl)amino-2-(3-trifluromethoxylphenyl)piperidine;
(2S,3S)-3-(2-isopropoxy-5-trifluoromethoxybenzyl)amino-2-phenylpiperidine;
(2S,3S)-3-(2-ethoxy-5-trifluoromethoxybenzyl)amino-2-phenylpiperidine;
(2S,3S)-3-(2-methoxy-5-trifluoromethoxybenzyl)amino-2-phenylpiperidine;
(2S,3S)-3-(5-tert-butyl-2-trifluoromethoxybenzyl)amino-2-phenylpiperidine;
2-(diphenylmethyl)-N-(2-methoxy-5-trifluoromethoxyphenyl)methyl-1-azabicyclo[2.2.2]octan-3-amine;
(2S,3S)-3-[5-chloro-2-(2,2,2-trifluoroethoxy)-benzyl]amino-2-phenylpiperidine;
(2S,3S)-3-(2-difluoromethoxy-5-trifluoromethoxybenzyl)amino-2-phenylpiperidine;
(2S,3S)-2-phenyl-3-[2-(2,2,2-trifluoroethoxybenzyl)]aminopiperidine;
(2S,3S)-2-phenyl-3-(2-trifluoromethoxybenzyl)aminopiperidine;
3-[N-(2-methoxy-5-trifluoromethoxybenzyl)-amino]-5,5-dimethyl-2-phenylpyrrolidine;
3-[N-(2-methoxy-5-trifluoromethoxybenzyl)-amino]-4,5-dimethyl-2-phenylpyrrolidine;
3-(2-cyclopropyloxy-5-trifluoromethoxybenzyl)amino-2-phenylpiperidine;
3-(2-cyclopropylmethoxy-5-trifluoromethoxybenzyl)amino-2-phenylpiperidine;
3-(2-difluoromethoxy-5-phenylbenzyl)amino-2-phenylpiperidine;
3-(5-cyclopropylmethoxy-2-difluoromethoxybenzyl)amino-2-phenylpiperidine;
3-(2-methoxybenzyl)amino-2-(3-trifluoromethoxyphenyl)piperidine;
3-(2-methoxy-5-trifluoromethoxybenzyl)amino-2-(3-trifluoromethoxyphenyl)piperidine;
2-phenyl-3-(5-n-propyl-2-trifluoromethoxybenzyl)amino-piperidine;
3-(5-isopropyl-2-trifluoromethoxybenzyl)amino-2-phenylpiperidine;
3-(5-ethyl-2-trifluoromethoxybenzyl)amino-2-phenylpiperidine;
3-(5-sec-butyl-2-trifluoromethoxybenzyl)amino-2-phenylpiperidine;
3-(5-difluoromethoxy-2-methoxybenzyl)amino-2-phenylpiperidine;
3-(2-methoxy-5-trifluoromethoxybenzyl)amino-2-phenylpyrrolidine;
3-(2-methoxy-5-trifluoromethoxybenzyl)amino-2-phenylhomopiperidine;
2-benzhydryl-3-(2-methoxy-5-trifluoromethoxy-benzyl)aminopyrrolidine;
2-benzhydryl-3-(2-methoxy-5-trifluoromethoxy-benzyl)aminohomopiperidine;
3-[2,5-bis-(2,2,2-trifluoroethoxy)benzyl]amino-2-phenylpiperidine;
2-phenyl-3-(3-trifluoromethoxybenzyl)aminopiperidine;
2-benzhydryl-3-(2-methoxy-5-trifluoromethoxybenzyl)aminopiperidine;
1-(5,6-difluorohexyl)-3-(2-methoxy-5-trifluoromethoxybenzyl)amino-2-phenylpiperidine;
1-(6-hydroxyhexyl)-3-(2-methoxy-5-trifluoromethoxybenzyl)amino-2-phenylpiperidine;
3-phenyl-4-(2-methoxy-5-trifluoromethoxybenzyl)amino-2-azabicydo[3.3.0]octane;
4-benzhydryl-5-(2-methoxy-5-trifluoromethoxybenzyl)amino-3-azabicyclo[4.1.0]heptane;
4-(2-methoxy-5-trifluoromethoxybenzyl)amino-3-phenyl-2-azabicyclo[4.4.0]decane;
2-phenyl-3-(2-methoxy-5-trifluoromethoxybenzyl)aminoquinuclidine;
8-benzhydryl-N-(2-methoxy-5-trifluoromethoxybenzyl)-9-azatricyclo[4.3.1.0^{4,9}]decan-7-amine;
9-benzhydryl-N-(2-methoxy-5-trifluoromethoxybenzyl)-10-azatricyclo[4.4.1.0^{5,10}]undecan-8-amine;
9-benzhydryl-N-(2-methoxy-5-trifluoromethoxybenzyl)-3-thia-10-azatricyclo[4.4.1.0^{5,10}]undecan-8-amine;
8-benzhydryl-N-(2-methoxy-5-trifluoromethoxybenzyl)-9-azatricyclo[4.3.1.0^{4,9}]decan-7-amine;
5,6-pentamethylene-2-benzhydryl-3-(2-methoxy-5-trifluoromethoxybenzyl)aminoquinuclidine;
5,6-trimethylene-2-benzhydryl-3-(2-methoxy-5-trifluoromethoxybenzyl)aminoquinuclidine;
9-benzhydryl-N-((2-methoxy-5-trifluoromethoxyphenyl)-methyl)-3-oxa-10-azatricyclo[4.4.1.0^{5,10}]undecan-3-amine;
8-benzhydryl-N-((2-methoxy-5-trifluoromethoxyphenyl)-methyl)-7-azatricyclo[4.4.1.0^{5,10}]undecan-9-amine; and
2-benzhydryl-N-((2-methoxy-5-trifluoromethoxyphenyl)-methyl)-1-azabicyclo[3.2.2]nonan-3-amine;
and pharmaceutically acceptable salts and solvates thereof.

5. The use as claimed in Claim 4 wherein the Substance P antagonist is (2S,3S)-3-(2-methoxy-5-trifluoromethoxybenzyl)amino-2-phenylpiperidine, or a pharmaceutically acceptable salt or solvate thereof.

6. The use of a pharmaceutically effective amount of a substance P antagonist for the preparation of a medicament for the prevention or treatment of emesis associated with a macrolide antibiotic in a subject in need of such prevention or treatment.

7. The use as claimed in Claim 6 wherein the subject is a companion animal.

8. The use as claimed in Claim 6 wherein the subject is a human.

9. The use as claimed in Claim 6 wherein the Substance P antagonist is selected from the group consisting of:
(2S,3S)-3-(5-tert-butyl-2-methoxybenzyl)amino-2-(3-trifluromethoxylphenyl)piperidine;
(2S,3S)-3-(2-isopropoxy-5-trifluoromethoxybenzyl)amino-2-phenylpiperidine;
(2S,3S)-3-(2-ethoxy-5-trifluoromethoxybenzyl)amino-2-phenylpiperidine;
(2S,3S)-3-(2-methoxy-5-trifluoromethoxybenzyl)amino-2-phenylpiperidine;
(2S,3S)-3-(5-tert-butyl-2-trifluoromethoxybenzyl)amino-2-phenylpiperidine;
2-(diphenylmethyl)-N-(2-methoxy-5-trifluoromethoxyphenyl)methyl-1-azabicyclo[2.2.2]octan-3-amine;
(2S,3S)-3-[5-chloro-2-(2,2,2-trifluoroethoxy)-benzyl]amino-2-phenylpiperidine;
(2S,3S)-3-(2-difluoromethoxy-5-trifluoromethoxybenzyl)amino-2-phenylpiperidine;
(2S,3S)-2-phenyl-3-[2-(2,2,2-trifluoroethoxybenzyl)]aminopiperidine;
(2S,3S)-2-phenyl-3-(2-trifluoromethoxybenzyl)aminopiperidine;
3-[N-(2-methoxy-5-trifluoromethoxybenzyl)-amino]-5,5-dimethyl-2-phenylpyrrolidine;
3-[N-(2-methoxy-5-trifluoromethoxybenzyl)-amino]-4,5-dimethyl-2-phenylpyrrolidine;
3-(2-cyclopropyloxy-5-trifluoromethoxybenzyl)amino-2-phenylpiperidine;
3-(2-cyclopropylmethoxy-5-trifluoromethoxybenzyl)amino-2-phenylpiperidine;
3-(2-difluoromethoxy-5-phenylbenzyl)amino-2-phenylpiperidine;
3-(5-cyclopropylmethoxy-2-difluoromethoxybenzyl)amino-2-phenylpiperidine;
3-(2-methoxybenzyl)amino-2-(3-trifluoromethoxyphenyl)piperidine;
3-(2-methoxy-5-trifluoromethoxybenzyl)amino-2-(3-trifluoromethoxyphenyl)piperidine;
2-phenyl-3-(5-n-propyl-2-trifluoromethoxybenzyl)amino-piperidine;
3-(5-isopropyl-2-trifluoromethoxybenzyl)amino-2-phenylpipeidine;
3-(5-ethyl-2-trifluoromethoxybenzyl)amino-2-phenylpiperidine;
3-(5-sec-butyl-2-trifluoromethoxybenzyl)amino-2-phenylpiperidine;
3-(5-difluoromethoxy-2-methoxybenzyl)amino-2-phenylpiperidine;
3-(2-methoxy-5-trifluoromethoxybenzyl)amino-2-phenylpyrrolidine;
3-(2-methoxy-5-trifluoromethoxybenzyl)amino-2-phenylhomopiperidine;
2-benzhydryl-3-(2-methoxy-5-trifluoromethoxy-benzyl)aminopyrrolidine;
2-benzhydryl-3-(2-methoxy-5-trifluoromethoxy-benzyl)aminohomopiperidine;
3-[2,5-bis-(2,2,2-trifluoroethoxy)benzyl]amino-2-phenylpiperidine;
2-phenyl-3-(3-trifluoromethoxybenzyl)aminopiperidine;
2-benzhydryl-3-(2-methoxy-5-trifluoromethoxybenzyl)aminopiperidine;
1-(5,6-difluorohexyl)-3-(2-methoxy-5-trifluoromethoxybenzyl)amino-2-phenylpiperidine;
1-(6-hydroxyhexyl)-3-(2-methoxy-5-trifluoromethoxybenzyl)amino-2-phenylpiperidine;
3-phenyl-4-(2-methoxy-5-trifluoromethoxybenzyl)amino-2-azabicyclo[3.3.0]octane;
4-benzhydryl-5-(2-methoxy-5-trifluoromethoxybenzyl)amino-3-azabicyclo[4.1.0]heptane;
4-(2-methoxy-5-trifluoromethoxybenzyl)amino-3-phenyl-2-azabicyclo[4.4.0]decane;
2-phenyl-3-(2-methoxy-5-trifluoromethoxybenzyl)aminoquinuclidine;
8-benzhydryl-N-(2-methoxy-5-trifluoromethoxybenzyl)-9-azatricyclo[4.3.1.0^{4,9}]decan-7-amine;
9-benzhydryl-N-(2-methoxy-5-trifluoromethoxybenzyl)-10-azatricyclo[4.4.1.0^{5,10}]undecan-8-amine;
9-benzhydryl-N-(2-methoxy-5-trifluoromethoxybenzyl)-3-thia-10-azatricyclo[4.4.1.0^{5,10}]undecan-8-amine;
8-benzhydryl-N-(2-methoxy-5-trifluoromethoxybenzyl)-9-azatricyclo[4.3.1.0^{4,9}]decan-7-amine;
5,6-pentamethylene-2-benzhydryl-3-(2-methoxy-5-trifluoromethoxybenzyl)aminoquinuclidine;
5,6-trimethylene-2-benzhydryl-3-(2-methoxy-5-trifluoromethoxybenzyl)aminoquinuclidine;
9-benzhydryl-N-((2-methoxy-5-trifluoromethoxyphenyl)-methyl)-3-oxa-10-azatricyclo[4.4.1.0^{5,10}]undecan-3-amine;
8-benzhydryl-N-((2-methoxy-5-trifluoromethoxyphenyl)-methyl)-7-azatricyclo[4.4.1.0^{5,10}]undecan-9-amine; and
2-benzhydryl-N-((2-methoxy-5-trifluoromethoxyphenyl)-methyl)-1-azabicyclo[3.2.2]nonan-3-amine;
and pharmaceutically acceptable salts and solvates thereof.

10. The use as claimed in Claim 9 wherein the Substance P antagonist is (2S,3S)-3-(2-methoxy-5-trifluoromethoxybenzyl)amino-2-phenylpiperidine, or a pharmaceutically acceptable salt or solvate thereof.

11. A pharmaceutical composition comprising a pharmaceutically effective amount of a macrolide antibiotic, a pharmaceutically effective amount of a Substance P antagonist, and optionally, a carrier.

12. The pharmaceutical composition of claim 11 wherein the carrier is an excipient.

## Patentansprüche

1. Verwendung einer pharmazeutisch wirksamen Menge eines Makrolidantibiotikums und einer pharmazeutisch wirksamen Menge eines Substanz-P-Antagonisten zur Herstellung eines Medikaments zur Behandlung von Krebs oder einer Bakterien- oder Protozoeninfektion bei einem eine derartige Behandlung benötigenden Säugersubjekt.

2. Verwendung gemäß Anspruch 1, wobei das Subjekt ein Begleitertier oder ein Mensch ist.

3. Verwendung gemäß Anspruch 1, wobei das Makrolidantibiotikum aus Erythromycin, Clarithromycin, Azithromycin, Josamycin oder Tylosin ausgewählt ist.

4. Verwendung gemäß Anspruch 1, wobei der Substanz-P-Antagonist ausgewählt ist aus der Gruppe von:
(2S,3S)-3-(5-tert.-Butyl-2-methoxybenzyl)amino-2-(3-trifluormethoxyphenyl)piperidin;
(2S,3S)-3-(2-Isopropoxy-5-trifluormethoxybenzyl)amino-2-phenylpiperidin;
(2S,3S)-3-(2-Ethoxy-5-trifluormethoxybenzyl)amino-2-phenylpiperidin;
(2S,3S)-3-(2-Methoxy-5-trifluormethoxybenzyl)amino-2-phenylpiperidin;
(2S,3S)-3-(5-tert.-Butyl-2-trifluormethoxybenzyl)amino-2-phenylpiperidin;
2-(Diphenylmethyl)-N-(2-methoxy-5-trifluormethoxyphenyl)-methyl-1-azabicyclo[2.2.2]octan-3-amin;
(2S,3S)-3-[5-Chlor-2-(2,2,2-trifluorethoxy)-benzyl]amino-2-phenylpiperidin;
(2S,3S)-3-(2-Difluormethoxy-5-trifluormethoxybenzyl)amino-2-phenylpiperidin;
(2S,3S)-2-Phenyl-3-[2-(2,2,2-trifluorethoxybenzyl)]aminopiperidin;
(2S,3S)-2-Phenyl-3-(2-trifluormethoxybenzyl)aminopiperidin;
3-[N-(2-Methoxy-5-trifluormethoxybenzyl)amino]-5,5-dimethyl-2-phenylpyrrolidin;
3-[N-(2-Methoxy-5-trifluormethoxybenzyl)amino]-4,5-dimethyl-2-phenylpyrrolidin;
3-(2-Cyclopropyloxy-5-trifluormethoxybenzyl)amino-2-phenylpiperidin;
3-(2-Cyclopropylmethoxy-5-trifluormethoxybenzyl)amino-2-phenylpiperidin;
3-(2-Difluormethoxy-5-phenylbenzyl)amino-2-phenylpiperidin;
3-(5-Cyclopropylmethoxy-2-difluormethoxybenzyl)amino-2-phenylpiperidin;
3-(2-Methoxybenzyl)amino-2-(3-trifluormethoxyphenyl)piperidin;
3-(2-Methoxy-5-trifluormethoxybenzyl)amino-2-(3-trifluormethoxyphenyl)piperidin;
2-Phenyl-3-(5-n-propyl-2-trifluormethoxybenzyl)amino-piperidin;
3-(5-Isopropyl-2-trifluormethoxybenzyl)amino-2-phenylpiperidin;
3-(5-Ethyl-2-trifluormethoxybenzyl)amino-2-phenylpiperidin;
3-(5-sek.-Butyl-2-trifluormethoxybenzyl)amino-2-phenylpiperidin;
3-(5-Difluormethoxy-2-methoxybenzyl)amino-2-phenylpiperidin;
3-(2-Methoxy-5-trifluormethoxybenzyl)amino-2-phenylpyrrolidin;
3-(2-Methoxy-5-trifluormethoxybenzyl)amino-2-phenylhomopiperidin;
2-Benzhydryl-3-(2-methoxy-5-trifluormethoxy-benzyl)amino-pyrrolidin;
2-Benzhydryl-3-(2-methoxy-5-trifluormethoxybenzyl)aminohomopiperidin;
3-[2,5-Bis-(2,2,2-trifluorethoxy)benzyl]amino-2-phenylpiperidin;
2-Phenyl-3-(3-trifluormethoxybenzyl)aminopiperidin;
2-Benzhydryl-3-(2-methoxy-5-trifluormethoxybenzyl)aminopiperidin;
1-(5,6-Difluorhexyl)-3-(2-methoxy-5-trifluormethoxybenzyl)amino-2-phenylpiperidin;
1-(6-Hydroxyhexyl)-3-(2-methoxy-5-trifluormethoxybenzyl)amino-2-phenylpiperidin;
3-Phenyl-4-(2-methoxy-5-trifluormethoxybenzyl)amino-2-azabicyclo[3.3.0]octan;
4-Benzhydryl-5-(2-methoxy-5-trifluormethoxybenzyl)amino-3-azabicyclo[4.1.0]heptan;
4-(2-Methoxy-5-trifluormethoxybenzyl)amino-3-phenyl-2-azabicyclo[4.4.0]decan;
2-Phenyl-3-(2-methoxy-5-trifluormethoxybenzyl)aminochinuclidin;
8-Benzhydryl-N-(2-methoxy-5-trifluormethoxybenzyl)-9-azatricyclo[4.3.1.04,9]decan-7-amin;
9-Benzhydryl-N-(2-methoxy-5-trifluormethoxybenzyl)-10-azatricyclo[4.4.1.05,10]undecan-8-amin;
9-Benzhydryl-N-(2-methoxy-5-trifluormethoxybenzyl)-3-thia-10-azatricyclo[4.4.1.05,10]undecan-8-amin;
8-Benzhydryl-N-(2-methoxy-5-trifluormethoxybenzyl)-9-azatricyclo[4.3.1.04,9]decan-7-amin;
5,6-Pentamethylen-2-benzhydryl-3-(2-methoxy-5-trifluormethoxybenzyl)aminochinuclidin;
5,6-Trimethylen-2-benzhydryl-3-(2-methoxy-5-trifluormethoxybenzyl)aminochinuclidin;
9-Benzhydryl-N-((2-methoxy-5-trifluormethoxyphenyl)-methyl)-3-oxa-10-azatricyclo[4.4.1.05,10]undecan-3-amin;
8-Benzhydryl-N-((2-methoxy-5-trifluormethoxyphenyl)-methyl)-7-azatricyclo[4.4.1.05,10]undecan-9-amin und
2-Benzhydryl-N-((2-methoxy-5-trifluormethoxyphenyl)-methyl)-1-azabicyclo[3.2.2]nonan-3-amin
und pharmazeutisch akzeptablen Salzen und Solvaten derselben.

5. Verwendung gemäß Anspruch 4, wobei der Substanz-P-Antagonist (2S,3S)-3-(2-Methoxy-5-trifluormethoxybenzyl)amino-2-phenylpiperidin oder ein pharmazeutisch akzeptables Salz oder Solvat desselben ist.

6. Verwendung einer pharmazeutisch wirksamen Menge eines Substanz-P-Antagonisten zur Herstellung eines Medikaments zur Prävention oder Behandlung von mit einem Makrolidantibiotikum in Verbindung stehender Emesis bei einem eine derartige Prävention oder Behandlung benötigenden Subjekt.

7. Verwendung gemäß Anspruch 6, wobei das Subjekt ein Begleitertier ist.

8. Verwendung gemäß Anspruch 8, wobei das Subjekt ein Mensch ist.

9. Verwendung gemäß Anspruch 6, wobei der Substanz-P-Antagonist ausgewählt ist aus der Gruppe von:
(2S,3S)-3-(5-tert.-Butyl-2-methoxybenzyl)amino-2-(3-trifluormethoxyphenyl)piperidin;
(2S,3S)-3-(2-Isopropoxy-5-trifluormethoxybenzyl)amino-2-phenylpiperidin;
(2S,3S)-3-(2-Ethoxy-5-trifluormethoxybenzyl)amino-2-phenylpiperidin;
(2S,3S)-3-(2-Methoxy-5-trifluormethoxybenzyl)amino-2-phenylpiperidin;
(2S,3S)-3-(5-tert.-Butyl-2-trifluormethoxybenzyl)amino-2-phenylpiperidin;
2-(Diphenylmethyl)-N-(2-methoxy-5-trifluormethoxyphenyl)-methyl-1-azabicyclo[2.2.2]octan-3-amin;
(2S,3S)-3-[5-Chlor-2-(2,2,2-trifluorethoxy)-benzyl]amino-2-phenylpiperidin;
(2S,3S)-3-(2-Difluormethoxy-5-trifluormethoxybenzyl)amino-2-phenylpiperidin;
(2S,3S)-2-Phenyl-3-[2-(2,2,2-trifluorethoxybenzyl)]aminopiperidin;
(2S,3S)-2-Phenyl-3-(2-trifluormethoxybenzyl)aminopiperidin;
3-[N-(2-Methoxy-5-trifluormethoxybenzyl)amino]-5,5-dimethyl-2-phenylpyrrolidin;
3-[N-(2-Methoxy-5-trifluormethoxybenzyl)amino]-4,5-dimethyl-2-phenylpyrrolidin;
3-(2-Cyclopropyloxy-5-trifluormethoxybenzyl)amino-2-phenylpiperidin;
3-(2-Cyclopropylmethoxy-5-trifluormethoxybenzyl)amino-2-phenylpiperidin;
3-(2-Difluormethoxy-5-phenylbenzyl)amino-2-phenylpiperidin;
3-(5-Cyclopropylmethoxy-2-difluormethoxybenzyl)amino-2-phenylpiperidin;
3-(2-Methoxybenzyl)amino-2-(3-trifluormethoxyphenyl)piperidin;
3-(2-Methoxy-5-trifluormethoxybenzyl)amino-2-(3-trifluormethoxyphenyl)piperidin;
2-Phenyl-3-(5-n-propyl-2-trifluormethoxybenzyl)amino-piperidin;
3-(5-Isopropyl-2-trifluormethoxybenzyl)amino-2-phenylpiperidin;
3-(5-Ethyl-2-trifluormethoxybenzyl)amino-2-phenylpiperidin;
3-(5-sek.-Butyl-2-trifluormethoxybenzyl)amino-2-phenylpiperidin;
3-(5-Difluormethoxy-2-methoxybenzyl)amino-2-phenylpiperidin;
3-(2-Methoxy-5-trifluormethoxybenzyl)amino-2-phenylpyrrolidin;
3-(2-Methoxy-5-trifluormethoxybenzyl)amino-2-phenylhomopiperidin;
2-Benzhydryl-3-(2-methoxy-5-trifluormethoxy-benzyl)amino-pyrrolidin;
2-Benzhydryl-3-(2-methoxy-5-trifluormethoxy-benzyl)aminohomopiperidin;
3-[2,5-Bis-(2,2,2-trifluorethoxy)benzyl]amino-2-phenylpiperidin;
2-Phenyl-3-(3-trifluormethoxybenzyl)aminopiperidin;
2-Benzhydryl-3-(2-methoxy-5-trifluormethoxybenzyl)aminopiperidin;
1-(5,6-Difluorhexyl)-3-(2-methoxy-5-trifluormethoxybenzyl)amino-2-phenylpiperidin;
1-(6-Hydroxyhexyl)-3-(2-methoxy-5-trifluormethoxybenzyl)amino-2-phenylpiperidin;
3-Phenyl-4-(2-methoxy-5-trifluormethoxybenzyl)amino-2-azabicyclo[3.3.0]octan;
4-Benzhydryl-5-(2-methoxy-5-trifluormethoxybenzyl)amino-3-azabicyclo[4.1.0]heptan;
4-(2-Methoxy-5-trifluormethoxybenzyl)amino-3-phenyl-2-azabicyclo[4.4.0]decan;
2-Phenyl-3-(2-methoxy-5-trifluormethoxybenzyl)aminochinuclidin;
8-Benzhydryl-N-(2-methoxy-5-trifluormethoxybenzyl)-9-azatricyclo[4.3.1.04,9]decan-7-amin;
9-Benzhydryl-N-(2-methoxy-5-trifluormethoxybenzyl)-10-azatricyclo[4.4.1.05,10]undecan-8-amin;
9-Benzhydryl-N-(2-methoxy-5-trifluormethoxybenzyl)-3-thia-10-azatricyclo[4.4.1.05,10]undecan-8-amin;
8-Benzhydryl-N-(2-methoxy-5-trifluormethoxybenzyl)-9-azatricyclo[4.3.1.04,9]decan-7-amin;
5,6-Pentamethylen-2-benzhydryl-3-(2-methoxy-5-trifluormethoxybenzyl)aminochinuclidin;
5,6-Trimethylen-2-benzhydryl-3-(2-methoxy-5-trifluormethoxybenzyl)aminochinuclidin;
9-Benzhydryl-N-((2-methoxy-5-trifluormethoxyphenyl)-methyl)-3-oxa-10-azatricyclo[4.4.1.05,10]undecan-3-amin;
8-Benzhydryl-N-((2-methoxy-5-trifluormethoxyphenyl)-methyl)-7-azatricyclo[4.4.1.05,10]undecan-9-amin und
2-Benzhydryl-N-((2-methoxy-5-trifluormethoxyphenyl)-methyl)-1-azabicyclo[3.2.2]nonan-3-amin
und pharmazeutisch akzeptablen Salzen und Solvaten derselben.

10. Verwendung gemäß Anspruch 9, wobei der Substanz-P-Antagonist (2S,3S)-3-(2-Methoxy-5-trifluormethoxybenzyl)amino-2-phenylpiperidin oder ein pharmazeutisch akzeptables Salz oder Solvat desselben ist.

11. Pharmazeutische Zusammensetzung, die eine pharmazeutisch wirksame Menge eines Makrolidantibiotikums, eine pharmazeutisch wirksame Menge eines Substanz-P-Antagonisten und op- tional einen Träger umfasst.

12. Pharmazeutische Zusammensetzung nach Anspruch 11, wobei der Träger ein Streckmittel ist.

## Revendications

1. Utilisation d'une quantité pharmaceutiquement efficace d'un antibiotique macrolide et d'une quantité pharmaceutiquement efficace d'un antagoniste de la Substance P pour la préparation d'un médicament pour le traitement du cancer ou d'une infection bactérienne ou à protozoaires chez un sujet mammifère nécessitant un tel traitement.

2. Utilisation selon la revendication 1, dans laquelle le sujet est un animal de compagnie ou un être humain.

3. Utilisation selon la revendication 1, dans laquelle l'antibiotique macrolide est sélectionné parmi l'érythromycine, la clarithromycine, l'azithromycine, la josamycine ou la tylosine.

4. Utilisation selon la revendication 1, dans laquelle l'antagoniste de la Substance P est sélectionné dans le groupe consistant en :
• la (2S,3S)-3-(5-tert-butyl-2-méthoxybenzyl)amino-2-(3-trifluorométhoxyphényl)pipéridine ;
• la (2S,3S)-3-(2-isopropoxy-5-trifluorométhoxybenzyl) amino-2-phénylpipéridine ;
• la (2S,3S)-3-(2-éthoxy-5-trifluorométhoxybenzyl)amino-2-phénylpipéridine ;
• la (2S,3S)-3-(2-méthoxy-5-trifluorométhoxybenzyl)amino-2-phénylpipéridine ;
• la (2S,3S)-3-(5-tert-butyl-2-trifluorométhoxybenzyl) amino-2-phénylpipéridine ;
• la 2-(diphénylméthyl)-N-(2-méthoxy-5-trifluorométhoxyphényl)méthyl-1-azabicyclo[2.2.2]octan-3-amine ;
• la (2S,3S)-3-[5-chloro-2-(2,2,2-trifluoroéthoxy)-benzyl] amino-2-phénylpipéridine ;
• la (2S,3S)-3-(2-difluorométhoxy-5-trifluorométhoxybenzyl)amino-2-phénylpipéridine ;
• la (2S,3S)-2-phényl-3-[2-(2,2,2-trifluoroéthoxybenzyl)]aminopipéridine ;
• la (2S,3S)-2-phényl-3-(2-trifluorométhoxybenzyl) aminopipéridine ;
• la 3-[N-(2-méthoxy-5-trifluorométhoxybenzyl)-amino]-5,5-diméthyl-2-phénylpyrrolidine ;
• la 3-[N-(2-méthoxy-5-trifluorométhoxybenzyl)-amino]-4,5-diméthyl-2-phénylpyrrolidine ;
• la 3-(2-cyclopropyloxy-5-trifluorométhoxybenzyl)amino-2-phénylpipéridine ;
• la 3-(2-cyclopropylméthoxy-5-trifluorométhoxybenzyl) amino-2-phénylpipéridine ;
• la 3-(2-difluorométhoxy-5-phénylbenzyl)amino-2-phénylpipéridine ;
• la 3-(5-cyclopropylméthoxy-2-difluorométhoxybenzyl)amino-2-phénylpipéridine ;
• la 3-(2-méthoxybenzyl)amino-2-(3-trifluorométhoxyphényl) pipéridine ;
• la 3-(2-méthoxy-5-trifluorométhoxybenzyl)amino-2-(3-trifluorométhoxyphényl)pipéridine ;
• la 2-phényl-3-(5-n-propyl-2-trifluorométhoxybenzyl)aminopipéridine ;
• la 3-(5-isopropyl-2-trifluorométhoxybenzyl)amino-2-phénylpipéridine ;
• la 3-(5-éthyl-2-trifluorométhoxybenzyl)amino-2-phénylpipéridine ;
• la 3-(5-sec-butyl-2-trifluorométhoxybenzyl)amino-2-phénylpipéridine ;
• la 3-(5-difluorométhoxy-2-méthoxybenzyl)amino-2-phénylpipéridine ;
• la 3-(2-méthoxy-5-trifluorométhoxybenzyl)amino-2-phénylpyrrolidine ;
• la 3-(2-méthoxy-5-trifluorométhoxybenzyl)amino-2-phénylhomopipéridine ;
• la 2-benzhydryl-3-(2-méthoxy-5-trifluorométhoxybenzyl) aminopyrrolidine ;
• la 2-benzhydryl-3-(2-méthoxy-5-trifluorométhoxybenzyl) aminohomopipéridine ;
• la 3-[2,5-bis-(2,2,2-trifluoroéthoxy)benzyl]amino-2-phénylpipéridine ;
• la 2-phényl-3-(3-trifluorométhoxybenzyl)aminopipéridine ;
• la 2-benzhydryl-3-(2-méthoxy-5-trifluorométhoxybenzyl) aminopipéridine ;
• la 1-(5,6-difluorohexyl)-3-(2-méthoxy-5-trifluorométhoxybenzyl)amino-2-phénylpipéridine ;
• la 1-(6-hydroxyhexyl)-3-(2-méthoxy-5-trifluorométhoxybenzyl)amino-2-phénylpipéridine ;
• le 3-phényl-4-(2-méthoxy-5-trifluorométhoxybenzyl)amino-2-azabicyclo[3.3.0]octane ;
• le 4-benzhydryl-5-(2-méthoxy-5-trifluorométhoxybenzyl) amino-3-azabicyclo[4.1.0]heptane ;
• le 4-(2-méthoxy-5-trifluorométhoxybenzyl)amino-3-phényl-2-azabicyclo[4.4.0]décane ;
• la 2-phényl-3-(2-méthoxy-5-trifluorométhoxybenzyl) aminoquinuclidine ;
• la 8-benzhydryl-N-(2-méthoxy-5-trifluorométhoxybenzyl)-9-azatricyclo[4.3.1.0^{4,9}]décan-7-amine ;
• la 9-benzhydryl-N-(2-méthoxy-5-trifluorométhoxybenzyl)-10-azatricyclo[4.4.1.0^{5,10}]undécan-8-amine ;
• la 9-benzhydryl-N-(2-méthoxy-5-trifluorométhoxybenzyl)-3-thia-10-azatricyclo[4.4.1.0^{5,10}]undécan-8-amine ;
• la 8-benzhydryl-N-(2-méthoxy-5-trifluorométhoxybenzyl)-9-azatricyclo[4.3.1.0^{4,9}]décan-7-amine ;
• la 5,6-pentaméthylène-2-benzhydryl-3-(2-méthoxy-5-trifluorométhoxybenzyl)aminoquinuclidine ;
• la 5,6-triméthylène-2-benzhydryl-3-(2-méthoxy-5-trifluorométhoxybenzyl)aminoquinuclidine ;
• la 9-benzhydryl-N-((2-méthoxy-5-trifluorométhoxyphényl)-méthyl)-3-oxa-10-azatricyclo[4.4.1.0^{5,10}]undécan-3-amine ;
• la 8-benzhydryl-N-((2-méthoxy-5-trifluorométhoxyphényl)-méthyl)-7-azatricyclo[4.4.1.0^{5,10}]undécan-9-amine ; et
• la 2-benzhydryl-N-((2-méthoxy-5-trifluorométhoxyphényl)-méthyl)-1-azabicyclo[3.2.2]nonan-3-amine ;
et leurs sels et solvates pharmaceutiquement acceptables.

5. Utilisation selon la revendication 4, dans laquelle l'antagoniste de la Substance P est la (2S,3S)-3-(2-méthoxy-5-trifluorométhoxybenzyl)amino-2-phénylpipéridine, ou un sel ou solvate pharmaceutiquement acceptable d'un tel composé.

6. Utilisation d'une quantité pharmaceutiquement efficace d'un antagoniste de la Substance P pour la préparation d'un médicament pour la prévention ou le traitement des vomissements associés à un antibiotique macrolide chez un sujet nécessitant une telle prévention ou un tel traitement.

7. Utilisation selon la revendication 6, dans laquelle le sujet est un animal de compagnie.

8. Utilisation selon la revendication 6, dans laquelle le sujet est un être humain.

9. Utilisation selon la revendication 6, dans laquelle l'antagoniste de la Substance P est sélectionné dans le groupe consistant en
• la (2S,3S)-3-(5-tert-butyl-2-méthoxybenzyl)amino-2-(3-trifluorométhoxyphényl)pipéridine ;
• la (2S,3S)-3-(2-isopropoxy-5-trifluorométhoxybenzyl) amino-2-phénylpipéridine ;
• la (2S,3S)-3-(2-éthoxy-5-trifluorométhoxybenzyl)amino-2-phénylpipéridine ;
• la (2S,3S)-3-(2-méthoxy-5-trifluorométhoxybenzyl)amino-2-phénylpipéridine ;
• la (2S,3S)-3-(5-tert-butyl-2-trifluorométhoxybenzyl) amino-2-phénylpipéridine ;
• la 2-(diphénylméthyl)-N-(2-méthoxy-5-trifluorométhoxyphényl)méthyl-1-azabicyclo[2.2.2]octan-3-amine ;
• la (2S,3S)-3-[5-chloro-2-(2,2,2-trifluoroéthoxy)-benzyl] amino-2-phénylpipéridine ;
• la (2S,3S)-3-(2-difluorométhoxy-5-trifluorométhoxybenzyl)amino-2-phénylpipéridine ;
• la (2S,3S)-2-phényl-3-[2-(2,2,2-trifluoroéthoxybenzyl)]aminopipéridine ;
• la (2S,3S)-2-phényl-3-(2-trifluorométhoxybenzyl) aminopipéridine ;
• la 3-[N-(2-méthoxy-5-trifluorométhoxybenzyl)-amino]-5,5-diméthyl-2-phénylpyrrolidine ;
• la 3-[N-(2-méthoxy-5-trifluorométhoxybenzyl)-amino]-4,5-diméthyl-2-phénylpyrrolidine ;
• la 3-(2-cyclopropyloxy-5-trifluorométhoxybenzyl)amino-2-phénylpipéridine ;
• la 3-(2-cyclopropylméthoxy-5-trifluorométhoxybenzyl) amino-2-phénylpipéridine ;
• la 3-(2-difluorométhoxy-5-phénylbenzyl)amino-2-phénylpipéridine ;
• la 3-(5-cyclopropylméthoxy-2-difluorométhoxybenzyl)amino-2-phénylpipéridine ;
• la 3-(2-méthoxybenzyl)amino-2-(3-trifluorométhoxyphényl) pipéridine ;
• la 3-(2-méthoxy-5-trifluorométhoxybenzyl)amino-2-(3-trifluorométhoxyphényl)pipéridine ;
• la 2-phényl-3-(5-n-propyl-2-trifluorométhoxybenzyl)aminopipéridine ;
• la 3-(5-isopropyl-2-trifluorométhoxybenzyl)amino-2-phénylpipéridine ;
• la 3-(5-éthyl-2-trifluorométhoxybenzyl)amino-2-phénylpipéridine ;
• la 3-(5-sec-butyl-2-trifluorométhoxybenzyl)amino-2-phénylpipéridine ;
• la 3-(5-difluorométhoxy-2-méthoxybenzyl)amino-2-phénylpipéridine ;
• la 3-(2-méthoxy-5-trifluorométhoxybenzyl)amino-2-phénylpyrrolidine ;
• la 3-(2-méthoxy-5-trifluorométhoxybenzyl)amino-2-phénylhomopipéridine ;
• la 2-benzhydryl-3-(2-méthoxy-5-trifluorométhoxybenzyl) aminopyrrolidine ;
• la 2-benzhydryl-3-(2-méthoxy-5-trifluorométhoxybenzyl) aminohomopipéridine ;
• la 3-[2,5-bis-(2,2,2-trifluoroéthoxy)benzyl]amino-2-phénylpipéridine ;
• la 2-phényl-3-(3-trifluorométhoxybenzyl)aminopipéridine ;
• la 2-benzhydryl-3-(2-méthoxy-5-trifluorométhoxybenzyl) aminopipéridine ;
• la 1-(5,6-difluorohexyl)-3-(2-méthoxy-5-trifluorométhoxybenzyl)amino-2-phénylpipéridine ;
• la 1-(6-hydroxyhexyl)-3-(2-méthoxy-5-trifluorométhoxybenzyl)amino-2-phénylpipéridine ;
• le 3-phényl-4-(2-méthoxy-5-trifluorométhoxybenzyl)amino-2-azabicyclo[3.3.0]octane ;
• le 4-benzhydryl-5-(2-méthoxy-5-trifluorométhoxybenzyl) amino-3-azabicyclo[4.1.0]heptane ;
• le 4-(2-méthoxy-5-trifluorométhoxybenzyl)amino-3-phényl-2-azabicyclo[4.4.0]décane ;
• la 2-phényl-3-(2-méthoxy-5-trifluorométhoxybenzyl) aminoquinuclidine ;
• la 8-benzhydryl-N-(2-méthoxy-5-trifluorométhoxybenzyl)-9-azatricyclo[4.3.1.0^{4,9}]décan-7-amine ;
• la 9-benzhydryl-N-(2-méthoxy-5-trifluorométhoxybenzyl)-10-azatricyclo[4.4.1.0^{5,10}]undécan-8-amine ;
• la 9-benzhydryl-N-(2-méthoxy-5-trifluorométhoxybenzyl)-3-thia-10-azatricyclo[4.4.1.0^{5,10}]undécan-8-amine ;
• la 8-benzhydryl-N-(2-méthoxy-5-trifluorométhoxybenzyl)-9-azatricyclo[4.3.1.0^{4,9}]décan-7-amine ;
• la 5,6-pentaméthylène-2-benzhydryl-3-(2-méthoxy-5-trifluorométhoxybenzyl)aminoquinuclidine ;
• la 5,6-triméthylène-2-benzhydryl-3-(2-méthoxy-5-trifluorométhoxybenzyl)aminoquinuclidine ;
• la 9-benzhydryl-N-((2-méthoxy-5-trifluorométhoxyphényl)-méthyl)-3-oxa-10-azatricyclo[4.4.1.0^{5,10}]undécan-3-amine ;
• la 8-benzhydryl-N-((2-méthoxy-5-trifluorométhoxyphényl)-méthyl)-7-azatricyclo[4.4.1.0^{5,10}]undécan-9-amine ; et
• la 2-benzhydryl-N-((2-méthoxy-5-trifluorométhoxyphényl)-méthyl)-1-azabicyclo[3.2.2]nonan-3-amine ;
et leurs sels et solvates pharmaceutiquement acceptables.

10. Utilisation selon la revendication 9, dans laquelle l'antagoniste de la Substance P est la (2S,3S)-3-(2-méthoxy-5-trifluorométhoxybenzyl)amino-2-phénylpipéridine, ou un sel ou solvate pharmaceutiquement acceptable d'un tel composé.

11. Composition pharmaceutique comprenant une quantité pharmaceutiquement efficace d'un antibiotique macrolide, une quantité pharmaceutiquement efficace d'un antagoniste de la Substance P, et, facultativement, un support.

12. Composition pharmaceutique selon la revendication 11, dans laquelle le support est un excipient.
